# EUROPÄISCHE PATENTSCHRIFT

(11) **EP 1 855 742 B1**
(45) Veröffentlichungstag und Bekanntmachung des Hinweises auf die Patenterteilung: **24.12.2008**
(21) Anmeldenummer: 06707178.7
(22) Anmeldetag: 22.02.2006
(51) Int. Cl.: A61M 5/315, A61M 5/24

(54) **DOSIERVORRICHTUNG ZUM EINSTELLEN FEINER DOSIERUNGEN**
DOSING DEVICE FOR SETTING FINE DOSES
DISPOSITIF DE DOSAGE POUR REGLER DES DOSAGES PRECIS

(30) Priorität: 22.02.2005 DE 102005008065; 24.05.2005 DE 102005023821
(43) Veröffentlichungstag der Anmeldung: 21.11.2007
(73) Patentinhaber: TecPharma Licensing AG, 3401 Burgdorf (CH)
(72) Erfinder: MOSER, Ulrich, CH-3412 Heimiswil (CH); SCHRUL, Christian, 3400 Burgdorf (CH); HIRSCHEL, Jürg, CH-5000 Aarau (CH)
(86) Internationale Anmeldenummer: PCT/EP2006/001616
(87) Internationale Veröffentlichungsnummer: WO 2006/089734

(56) Entgegenhaltungen:
- FR-A- 2 612 782
- US-A1- 2002 123 717
- US-A1- 2003 089 743
- US-A1- 2004 236 285
- US-B1- 6 235 004

## Beschreibung

Die vorliegende Erfindung bezieht sich auf eine Dosiervorrichtung für eine Injektionsvorrichtung zur dosierten Verabreichung eines injizierbaren Produkts mit einer Gewindestange mit einem umlaufenden eine Zahnung tragenden Gewinde, wobei mit der vorliegenden Dosiervorrichtung zum Beispiel feine Dosismengen eines injizierbaren Produkts eingestellt werden können, die üblicherweise Gewindestangen mit kleineren Abständen zwischen den Zähnen der Zahnung erfordern.

Die US 6,235,004 bezieht sich auf eine Injektionsspritze zum Verabreichen einstellbarer Dosen einer Medizin aus einer Kartusche, mit einem Gehäuse, welches eine Halterung zum Umfassen der Kartusche mit Medizin enthält, mit einer Kolbenstange mit einem nichtkreisförmigen Querschnitt und einem Außengewinde, mit einem Kolbenstangenantrieb, mit einer unidirektionalen Kupplung mit einem kranzförmigen Ring von inneren Sperrnuten, deren kranzförmiger Ring in das Gehäuse integriert sein kann, und einer Sperre, welche zumindest ein Paar federnder Arme aufweist, wobei jeder der Arme ein freies Ende besitzt.

Die US 2004/236285 offenbart eine Dosiervorrichtung bestehend unter anderem aus einem Gehäuse, auf dessen Innenseite eine gleichmäßige rampenartige Verzahnung in Längsrichtung angeordnet ist, und zwei Drehbegrenzungselementen mit jeweils einem Rastelement. Die Drehbegrenzungselemente sind leicht versetzt angeordnet, so daß, wenn das eine Rastelement in der Verzahnung verrastet, das andere Rastelement eine Zwischenposition auf der Rampe der Verzahnung einnimmt. Das Gewinde der Gewindestange trägt keine Verzahnung und auch keine Rastelemente für die Dosierungseinstellung.

Bekannte Dosiervorrichtungen besitzen zum feinen Dosieren eines injizierbaren Produkts eine Gewindestange mit einer hohen Anzahl nah aneinanderliegender Zähne einer Zahnung. Solche Gewindestangen sind jedoch fertigungstechnisch nur mit erheblichem Aufwand herzustellen.

Es ist eine Aufgabe der vorliegenden Erfindung die Nachteile aus dem Stand der Technik zu überwinden. Es ist weiter eine Aufgabe der vorliegenden Erfindung eine einfach zu fertigende Dosiervorrichtung für eine Injektionsvorrichtung vorzuschlagen, mittels welcher kleinere Dosiermengen eines injizierbaren Produkts eingestellt und verabreicht werden können.

Diese Aufgabe wird durch den Gegenstand der unabhängigen Ansprüche gelöst. Vorteilhafte Ausführungsformen ergeben sich aus den abhängigen Ansprüchen.

Die erfindungsgemäße Dosiervorrichtung wird in einer Injektionsvorrichtung zum dosierten Abgeben eines injizierbaren Produkts verwendet, um möglichst feine Dosierungen einstellen zu können und anschließend verabreichen zu können. Eine solche Injektionsvorrichtung weist ein Gehäuse oder Gehäuseelement oder Gehäuseteil auf, in welchem vorzugsweise eine Einstellhülse zum einmaligen Vordosieren oder Einstellen der Injektionsvorrichtung oder zum mehrmaligen Einstellen verschiedener Dosismengen drehbar angeordnet sein kann. Innerhalb der Einstellhülse kann eine Drehhülse drehbar gelagert werden, welche ein Außengewinde aufweisen kann, das in ein Innengewinde der Injektionsvorrichtung oder des Gehäuseteils der Injektionsvorrichtung eingreifen kann. Insbesondere kann durch Drehen eines vorzugsweise mit der Drehhülse verbundenen Drehknopfes oder Einstellknopfes eine Dosismenge eingestellt werden. Durch den Drehvorgang des Drehknopfes zum Einstellen der Dosismenge kann der Dosierknopf mitsamt der Drehhülse entgegen der Ausschüttrichtung des injizierbaren Produkts gegenüber der Einstellhülse verschoben werden. Auch kann zunächst durch Drehen der Einstellhülse eine Dosismenge eingestellt werden und durch Ziehen des Drehknopfes der Drehknopf mitsamt der Drehhülse entgegen der Ausschüttrichtung des injizierbaren Produkts aufgezogen werden. Zum Ausschütten einer voreingestellten Dosis kann der Drehknopf in Ausschüttrichtung gedrückt werden, wodurch die zum Beispiel drehbar in dem Drehknopf gelagerte Drehhülse in die Injektionsvorrichtung eingedreht werden kann, so dass eine Drehbewegung einer Gewindestange erzeugt werden kann, welche einen zum Beispiel an der Vorderseite der Gewindestange anliegenden Stopfen in eine Ampulle einschieben kann, um eine in der Ampulle enthaltene Substanz zu verdrängen und so die voreingestellte Dosis abzugeben.

Die erfindungsgemäße Dosiervorrichtung zur dosierten Verabreichung eines injizierbaren Produkts aus einer Injektionsvorrichtung umfasst eine Gewindestange mit einem um die Gewindestange laufenden Gewinde, welches eine Zahnung trägt oder an welchem eine Zahnung ausgebildet ist. Die Zahnung weist eine Vielzahl an Zähnen auf, welche entlang des Gewindegangs des Gewindes in einem in Umfangsrichtung vorzugsweise konstanten ersten Abstand voneinander angeordnet sind. Auch können mehrere jeweils eine Zahnung tragende Gewinde auf der Gewindestange angeordnet sein, wobei die Zähne der Zahnung eines Gewindes entlang des jeweiligen Gewindeganges in Umfangsrichtung der Gewindestange einen vorzugsweise konstanten ersten Abstand voneinander aufweisen.

Weiter weist die Dosiervorrichtung ein erstes Drehbegrenzungselement auf, welches mit der Injektionsvorrichtung oder einem Gehäuseteil der Injektionsvorrichtung vorzugsweise fest verbunden ist. Das Drehbegrenzungselement weist mindestens ein, insbesondere ein, zwei, drei oder vier oder mehr Rastelemente auf, welche ausgebildet sind, um mit der Zahnung der Gewindestange verrasten zu können. Verrasten ein oder mehr Rastelemente mit der Zahnung, so kann die Gewindestange relativ zu dem Rastelement vorzugsweise nur in eine Richtung gedreht oder eingedreht werden, wie zum Beispiel im oder gegen den Uhrzeigersinn gedreht oder in Richtung des distalen Endes der Injektionsvorrichtung hineingedreht werden, da eine Drehung in eine weitere Richtung durch eine Kraftübertragung des Rastelements auf die Gewindestange oder durch ein Halten der Gewindestange durch das Rastelement verhindert wird.

Vorzugsweise sind die Zähne der Zahnung als unelastische sägezahnförmige Nocken ausgebildet, welche eine senkrecht zu der Gewindestange verlaufende Anschlagsflanke und eine schräg zu der Gewindestange verlaufende Gleitflanke aufweisen können. Verrastet ein Rastelement mit solchen Nocken ist eine Drehung der Gewindestange nur in die Richtung möglich, über welche das Rastelement entlang der schrägen Gleitflanke abgleiten kann, indem zum Beispiel die relativ zu der Gewindestange vorzugsweise radial bewegbaren Rastelemente der Drehbegrenzungselemente eine radiale Bewegung entsprechend oder entlang der Steigung der Gleitflanke ausführen. Eine Drehung in die entgegengesetzte Richtung ist bei einer Verrastung oder bei einem Einrasten nicht möglich, da das Rastelement an der senkrechten Anschlagsflanke des Nockens anliegt und somit eine Gegenkraft entgegen der beabsichtigten Drehung oder Drehrichtung ausüben kann, die Drehung somit verhindern und die Zahnstange festhalten kann.

Die erfindungsgemäße Dosiervorrichtung umfasst weiter ein vorzugsweise fest mit der Drehhülse der Injektionsvorrichtung verbundenes zweites Drehbegrenzungselement, welches mindestens eins, vorzugsweise eins, zwei, drei, vier oder mehr Rastelemente umfasst.

Bevorzugt umfassen das erste und das zweite Drehbegrenzungselement jeweils zwei oder drei Rastelemente. Das mindestens eine Rastelement des zweiten Drehbegrenzungselements kann genauso ausgebildet sein wie das mindestens eine Rastelement des ersten Drehbegrenzungselements oder kann anders ausgebildet sein. Das mindestens eine Rastelement des zweiten Drehbegrenzungselements ist so ausgebildet, dass ein Verrasten mit der Zahnung der Gewindestange, wie zuvor beschrieben, ermöglicht wird. Das zweite Drehbegrenzungselement ist vorzugsweise in Längsrichtung der Gewindestange beabstandet von dem ersten Drehbegrenzungselement angeordnet.

Durch Drehen oder Ziehen eines mit der Drehhülse verbundenen oder gekoppelten Dosierknopfs kann eine Dosismenge eingestellt werden, indem das mit der Drehhülse verbundene zweite Drehbegrenzungselement relativ zu der Gewindestange in proximaler Richtung oder entgegen der Ausschüttrichtung des injizierbaren Produkts bewegt wird, während die Gewindestange vorzugsweise aufgrund der Verrastung mit dem ersten Drehbegrenzungselement in Ruhe gehalten wird. Durch Drücken des Dosierknopfes führt die Gewindestange aufgrund der Kraftübertragung des Dosierknopfes auf die Drehhülse und der Kraftübertragung des mit der Drehhülse verbundenen zweiten Drehbegrenzungselements auf die Gewindestange eine Drehbewegung und eine translatorische Bewegung relativ zu dem ersten Drehbegrenzungselement aus. Dadurch wird ein Ausschütten des injizierbaren Produkts mit der eingestellten Dosis erreicht.

Um feine Dosismengen einzustellen ist das mindestens eine Rastelement des ersten oder zweiten Drehbegrenzungselements so angeordnet oder ausgebildet, dass bei einer Dosismengeneinstellung, welche durch eine Drehung mindestens eines der Drehbewegungselemente, vorzugsweise des zweiten Drehbegrenzungselement, relativ zu der Gewindestange von weniger als dem ersten Abstand zwischen den Zähnen der Zahnung in Umfangsrichtung der Gewindestange hervorgerufen wird, das mindestens eine Rastelement in einer Zwischenposition verrasten kann. Der Abstand der Zwischenposition von der Ausgangsposition oder der zum Erreichen der Zwischenposition zurückgelegte Drehweg oder Weg mindestens eines oder mehrerer der Drehbegrenzungselemente relativ zu der Gewindestange ist kleiner als der erste Abstand zwischen den Zähnen der Zahnung in Umfangsrichtung. Bei herkömmlichen Dosiervorrichtungen würde bei einer Drehbewegung, welche kleiner ist als der Abstand zwischen zwei benachbarten Zähnen der Zahnung die Gewindestange in die ursprüngliche Position oder Ausgangsposition zurückgleiten, so dass es zu keiner Einstellung einer Dosis kommen würde. Nur bei einem Drehweg, welcher mindestens dem Abstand zweier benachbarter Zähne entspricht, wird bei herkömmlichen Dosiervorrichtungen eine minimal mögliche Dosis eingestellt. Mit der erfindungsgemäßen Dosiervorrichtung können durch das Einstellen von Zwischenpositionen Dosierungen erreicht werden, die kleiner sind als die bei einer Drehung von einem Zahn zu einem benachbarten Zahn erreichbaren oder einstellbaren Dosismengen.

Die in Längsrichtung der Gewindestange benachbarten Zähne, also die Zähne, die entlang der Längsachse auf einer parallel zur Längsachse verlaufenden Linie oder auf einer ZickZack-Linie entlang der Längsachse verlaufen, können in Umfangsrichtung der Gewindestange an gleicher Position angeordnet sein, so dass die Zähne der Zahnung entlang der Längsachse auf zu der Längsachse der Gewindestange parallelen Linien der Gewindestange liegen. Vorzugsweise sind die in Längsrichtung der Gewindestange zum Beispiel schräg zur Längsachse der Gewindestange benachbarten Zähne in Umfangsrichtung versetzt zueinander angeordnet, so dass es keine in Längsrichtung direkt benachbarten Zähne gibt, welche entlang einer Parallelen zur Längsachse verlaufen. Bevorzugt entspricht der Abstand der schräg in Längsrichtung benachbarten Zähne in Umfangsrichtung der Hälfte des ersten Abstands zwischen in Längsrichtung an einer Position angeordneten Zähnen. Jedoch kann dieser Abstand auch ein Drittel oder ein Viertel des ersten Abstands betragen. Sind zum Beispiel n (n ∈ N) Rastelemente an einem oder beiden Drehbegrenzungselementen angeordnet, so können die schräg zur Längsachse der Gewindestange benachbarten Zähne in Umfangsrichtung einen Abstand voneinander aufweisen, welcher dem n-ten Teil des ersten Abstands zwischen den Zähnen entspricht, welche in Längsrichtung an einer Position und in Umfangsrichtung nebeneinander angeordnet sind. Mit der Anordnung schräg benachbarter Zähne in Längsrichtung der Gewindestange können bei einer geeigneten Wahl der Drehbegrenzungselemente oder Rastelemente oder bei einer geeigneten Ausbildung oder einer geeigneten Anordnung der Drehbegrenzungselemente oder Rastelemente feine oder feinere Dosismengen eingestellt werden.

Insbesondere kann eines oder können beide der Drehbegrenzungselemente zwei oder mehr Rastelemente aufweisen, welche entlang des Umfangs der Gewindestange angeordnet und in die Zahnung eingreifen können. Zum Beispiel können zwei Rastelemente sich nahezu gegenüberliegen, so dass die Abweichung von einem genauen oder exakten Gegenüberliegen durch einen Versatz beschrieben werden kann. Die zwei Rastelemente können voneinander entlang des Umfangs einen Abstand aufweisen, welcher nahezu dem halben Umfang der Gewindestange entspricht. Die Abweichung von dem exakten halben Umfang der Gewindestange kann durch einen Versatz der Rastelemente zueinander beschrieben werden. Insbesondere weisen die Eingriffsspitzen der Rastelemente, welche beim Verrasten der Rastelemente mit der Zahnung direkt auf der Oberfläche oder dem Umfang der Gewindestange liegen, einen solchen Abstand oder Versatz voneinander auf. Der Versatz zwischen den Rastelementen beträgt dabei bevorzugt die Hälfte des ersten Abstands zwischen den Zähnen der Zahnung. Auch können drei Rastelemente entlang des Umfangs der Gewindestange angeordnet sein, welche zum Beispiel nahezu einen Abstand voneinander haben, welcher einem Drittel des Umfangs der Gewindestange entspricht. Der jeweilige Versatz der drei Rastelemente von der genauen Drittelung beträgt dabei vorzugsweise ein Drittel des ersten Abstands der Zähne der Zahnung voneinander. Sind zum Beispiel n Rastelemente an einem Drehbegrenzungselement entlang des Umfangs der Gewindestange angeordnet, so entspricht der Versatz in Umfangsrichtung der Gewindestange der benachbarten Rastelemente zueinander vorzugsweise dem n-ten Teil des ersten Abstands zwischen den Zähnen. Durch den Versatz der Rastelemente zueinander können die Rastelemente abwechselnd mit der Zahnung verrasten, um eine Dosiseinstellung oder Abgabe einer Dosis zu gewährleisten. Durch die abwechselnde Verrastung können kleinere Dosismengen eingestellt und abgegeben werden. Beträgt bei der Verwendung von 2 Rastelementen pro Drehbegrenzungselement der Versatz in Umfangsrichtung die Hälfte des ersten Abstands zwischen den Zähnen, so kann eine halb so große Dosis eingestellt werden wie ohne einen solchen Versatz.

Auch kann ein Rastelement oder können mehrere Rastelemente zum Beispiel an Stelle einer einzelnen Eingriffspitze eine Vielzahl von Eingriffselementen, wie zwei, drei, vier oder mehr Eingriffselemente aufweisen, welche bevorzugt einen konstanten Eingriffselementabstand voneinander aufweisen. Dieser Eingriffselementabstand kann bei der Verwendung von zwei Eingriffselementen pro Rastelement zum Beispiel der Hälfte des ersten Abstands der Zähne zueinander oder bei der Verwendung von drei Eingriffselementen pro Rastelement einem Drittel des ersten Abstands entsprechen. Werden zum Beispiel n Eingriffselemente an einem Rastelement angeordnet entspricht der Abstand zwischen den Eingriffselementen bevorzugt dem n-ten Teil des ersten Abstands zwischen den Zähnen der Zahnung. Durch die Ausbildung zweier oder mehrerer Eingriffselemente an einem oder mehreren Rastelementen können, wie zuvor im Bezug auf den Versatz der Rastelemente zueinander beschrieben, feinere Dosierungen mit der Dosiervorrichtung eingestellt und mit der Injektionsvorrichtung verabreicht werden.

Vorzugsweise können zwei oder mehr in Längsrichtung der Gewindestange benachbarte Zähne einen zweiten Abstand in Längsrichtung voneinander aufweisen oder es können alle benachbarten Zähne der Zahnung entlang der Längsachse der Gewindestange einen zweiten konstanten Abstand voneinander aufweisen. Die Rastelemente eines Drehbegrenzungselements oder beider Drehbegrenzungselemente können in Längsrichtung der Gewindestange an gleicher Position angeordnet sein oder können in Längsrichtung der Gewindestange beabstandet voneinander angeordnet sein. Bevorzugt entspricht der Abstand in Längsrichtung zwischen den Rastelementen eines Drehbegrenzungselements dem zweiten Abstand der in Längsrichtung benachbarten Zähne voneinander, so dass die Zahnung entlang der Rastelemente geführt werden kann und die Rastelemente an jedem beliebigen Zahn der Zahnung verrasten oder eingreifen können.

Vorzugsweise kann ein Rastelement oder können mehrere Rastelemente breiter als ein Zahn der Zahnung ausgebildet sein und sich bevorzugt entlang zwei, drei, vier oder mehr Zähnen entlang der Längsachse der Gewindestange erstrecken. Sind zum Beispiel die in Längsrichtung benachbarten Zähne in Umfangsrichtung an gleicher Position angeordnet, erstrecken sich somit die benachbarten Zähne in Längsrichtung auf einer Parallelen zur Längsachse der Gewindestange, kann ein Rastelement sich entlang zwei, drei, vier oder mehr Zähnen erstrecken und mit jedem der auf der Parallelen angeordneten Zähne verrasten. Sind die Zähne der Zahnung in Längsrichtung schräg benachbart oder sind die in Längsrichtung benachbarten Zähne in Umfangsrichtung versetzt voneinander angeordnet, so kann sich das breite Rastelement, entlang zwei, drei oder vier Zähnen erstrecken, jedoch zum Beispiel nur mit dem ersten oder mit dem ersten und dritten Zahn verrasten. Wird das breite Rastelement relativ zu der Gewindestange, zum Beispiel zum Einstellen oder Abgeben einer Dosis, bewegt oder gedreht, so kann das breite Rastelement zum Beispiel mit dem zweiten oder mit dem zweiten und vierten Zahn verrasten und mit den übrigen Zähnen entrasten, so dass kleinere oder feinere Drehbewegungen der Gewindestange relativ zu dem Rastelement bis zu einer erneuten Verrastung durchgeführt werden können, was zu einer Verfeinerung der Dosierung führt.

Durch die verschiedenen Möglichkeiten einer verfeinerten Dosiseinstellung wird vorzugsweise erreicht, dass in einem ersten Zustand, wie einem Ausgangszustand der Dosiervorrichtung die Anschlagsflanke eines ersten Rastelements an einem Gegenanschlag eines Zahns der Zahnung anliegt, um zum Beispiel eine Drehung der Gewindestange durchzuführen oder zu verhindern. In diesem ersten Zustand liegt vorzugsweise die Anschlagsflanke eines zweiten Rastelements desselben Drehbegrenzungselements, nicht an einem Gegenanschlag, zum an einer Gleitflanke eines Zahns der Zahnung an, so dass die Gewindestange zum Beispiel entlang des Zahns bewegen kann. Wurde die Zahnstange, zum Beispiel nach der Durchführung eines Aufziehvorgangs, in einen zweiten Zustand bewegt, liegt vorzugsweise die Anschlagsflanke des ersten Rastelements nicht an einem Gegenanschlag eines Zahns an, sondern die Anschlagsflanke des zweiten Rastelements liegt an einem Gegenschlag eines Zahns der Zahnung an.

Vorzugsweise ist der erste Abstand der Zähne der Zahnung in Umfangsrichtung kleiner als der zweite Abstand der benachbarten Zähne in Längsrichtung der Gewindestange, wobei auch der zweite Abstand kleiner als der erste Abstand sein kann.

Vorzugsweise erstreckt sich die Zahnung entlang des Gewindes der Gewindestange über die gesamte Länge der Gewindestange. Auch kann die Zahnung nur abschnittsweise ausgebildet sein, zum Beispiel in aneinander angrenzenden oder beabstandeten Abschnitten ausgebildet sein, wobei die Zahnung, wie zum Beispiel die Abstände der Zähne voneinander, in den Abschnitten gleich oder unterschiedlich ausgebildet sein kann.

Gemäß einem weiteren Aspekt der Erfindung weist die Dosiervorrichtung zur dosierten Verabreichung eines injizierbaren Produkts aus einer Injektionsvorrichtung eine Gewindestange auf, welche anstelle einer Zahnung eine entlang des Umfangs der Gewindestange umlaufende gewindeförmige Führungsrille umfasst, wobei entlang der Längsachse der Gewindestange zwischen den Abschnitten der Führungsrille eine Vielzahl von Eingriffselementen ausgebildet ist. Insbesondere sind die Eingriffselemente auf der Gewindestange so angeordnet, dass zwischen jedem Führungsrillenabschnitt und dem in Längsrichtung benachbarten Führungsrillenabschnitt ein Eingriffselement angeordnet ist. Insbesondere können die Eingriffselemente auch so auf der Gewindestange angeordnet sein, dass sie entlang der Längsachse der Gewindestange auf einer Parallelen zur Längsachse der Gewindestange angeordnet sind.

Weiter weist diese Dosiervorrichtung ein mit der Injektionsvorrichtung oder einem Gehäuseteil der Injektionsvorrichtung verbundenes erstes Drehbegrenzungselement auf, welches die Gewindestange vorzugsweise vollständig umgibt und welches vorzugsweise zylinderförmig oder als ein offener Zylinder ausgebildet ist, auf dessen Innenseite ein oder mehrere Führungselemente und eine Zahnung ausgebildet sind. Das Führungselement oder die Führungselemente sind so ausgebildet, dass sie ein Eingreifen in die Führungsrille der Gewindestange ermöglichen, so dass das Drehbegrenzungselement entlang der Gewindestange oder entlang der Führungsrille der Gewindestange bewegt werden kann und eine Drehbewegung und eine translatorische Bewegung relativ zu der Gewindestange ausführen kann. Die auf der Innenseite des Drehbegrenzungselements ausgebildete Zahnung ist so ausgebildet oder angeordnet, dass sie mit den auf der Gewindestange angeordneten Eingriffselementen verrasten kann und eine Kraft auf die Gewindestange, zum Beispiel zum Halten oder Bewegen der Gewindestange, übertragen kann. Die in Längsrichtung der Gewindestange an gleicher Position angeordneten, in Umfangsrichtung der Gewindestange benachbarten Zähne der Zahnung weisen einen ersten Abstand in Umfangsrichtung voneinander auf.

Weiter umfasst die Dosiervorrichtung ein mit einer Drehhülse der Injektionsvorrichtung verbundenes zweites Drehbegrenzungselement auf, welches die Gewindestange, insbesondere vollständig, umgibt. Das mit der Drehhülse verbundene zweite Drehbegrenzungselement ist bevorzugt genauso wie das mit der Injektionsvorrichtung verbundene erste Drehbegrenzungselement ausgebildet und weist auf der Innenseite ein oder mehrere Führungselemente auf, welche in die Führungsrille der Zahnstange eingreifen können, und weist auf der Innenseite eine Zahnung auf, welche mit den Eingriffselementen der Gewindestange verrasten kann. Die in Umfangsrichtung der Gewindestange benachbarten Zähne der Zahnung besitzen einen ersten Abstand voneinander.

Um eine feine Dosierung zu ermöglichen ist die Zahnung der Drehbegrenzungselemente so angeordnet oder ausgebildet, dass bei einer Drehung der Gewindestange relativ zu einem oder beiden der Drehbegrenzungselemente um weniger als den ersten Abstand der Zähne der Zahnung voneinander mindestens eines der Eingriffselemente der Gewindestange in einer Zwischenposition mit der Zahnung verrasten kann. Der bis zum Verrasten in der Zwischenposition zurückgelegte Weg ist dabei kleiner als der bis zum Verrasten zwischen in Umfangsrichtung benachbarten, in Längsrichtung an gleicher Position angeordneten Zähnen zurückgelegte Weg. Damit können feinere Dosierungen erreicht werden, als bei herkömmlichen Dosiervorrichtungen, welche lediglich ein Einrasten zwischen den Umfangsrichtungen benachbarten Zähnen gewährleisten.

Vorzugsweise kann die Zahnung auf der Innenseite des ersten und zweiten Drehbegrenzungselements aus zwei, drei, vier, fünf, sechs oder mehr in Längsrichtung des Drehbegrenzungselements versetzt zueinander angeordneten Teilzahnungen bestehen. Die in Längsrichtung benachbarten Zähne der benachbarten Teilzahnungen sind vorzugsweise in Umfangsrichtung des Begrenzungselements versetzt zueinander angeordnet und vorzugsweise um einen Abstand voneinander angeordnet, welcher der Hälfte oder einem Drittel oder einem Viertel oder einem Fünftel oder einem Sechstel des ersten Abstands zwischen zwei in Umfangsrichtung benachbarten Zähnen entspricht, welche in Längsrichtung des Drehbegrenzungselements an gleicher Stelle angeordnet sind. Auch können n in Längsrichtung des Drehbegrenzungselements versetzt zueinander angeordnete Teilzahnungen auf dem Drehbegrenzungselement angeordnet sein. Die in Längsrichtung des Drehbegrenzungselements benachbarten Zähne der benachbarten Teilzahnungen sind in Umfangsrichtung vorzugsweise um den n-ten Teil des ersten Abstands beabstandet voneinander angeordnet.

Die Zahnung kann über die gesamte Länge des Drehbegrenzungselements auf der Innenseite des Drehbegrenzungselements ausgebildet sein oder kann abschnittsweise ausgebildet sein. Beispielsweise können die Zähne der Zahnung in einem ersten entlang der Längsachse des Drehbegrenzungselements ausgebildeten Abschnitt einen konstanten Abstand in Umfangsrichtung des Drehbegrenzungselements voneinander aufweisen und in einem benachbarten oder angrenzenden zweiten entlang der Längsachse des Drehbegrenzungselements ausgebildeten Abschnitt jeweils einen weiteren von dem Abstand in dem ersten Abschnitt verschiedenen konstanten Abstand in Umfangsrichtung des Drehbegrenzungselements voneinander aufweisen.

Die Erfindung wird nachfolgend anhand bevorzugter Ausführungsbeispiel beschrieben. Es zeigen:
- Figur 1a: einen Ausschnitt einer ersten Ausführungsform der Dosiervorrichtung der vorliegenden Erfindung;
- Figur 1b: eine Frontansicht der ersten Ausführungsform der Dosiervorrichtung der vorliegenden Erfindung;
- Figur 1c: eine Seitenansicht der Gewindestange der ersten Ausführungsform der Dosiervorrichtung der vorliegenden Erfindung;
- Figur 1d: eine weitere Ansicht der Gewindestange der ersten Ausführungsform der Dosiervorrichtung der vorliegenden Erfindung;
- Figur 1e: eine Ansicht eines Drehbegrenzungselements der ersten Ausführungsform der Dosiervorrichtung der vorliegenden Erfindung;
- Figur 1f: eine weitere Ansicht des Drehbegrenzungselements der ersten Ausführungs-form der Dosiervorrichtung der vorliegenden Erfindung;
- Figur 2a: einen Ausschnitt einer zweiten Ausführungsform der Dosiervorrichtung der vorliegenden Erfindung;
- Figur 2b: eine Frontansicht der zweiten Ausführungsform der Dosiervorrichtung der vorliegenden Erfindung;
- Figur 2c: eine Seitenansicht der Gewindestange der zweiten Ausführungsform der Dosiervorrichtung der vorliegenden Erfindung;
- Figur 2d: eine weitere Ansicht der Gewindestange der zweiten Ausführungsform der Dosiervorrichtung der vorliegenden Erfindung;
- Figur 2e: eine Ansicht eines Drehbegrenzungselements der zweiten Ausführungsform der Dosiervorrichtung der vorliegenden Erfindung;
- Figur 2f: eine weitere Ansicht des Drehbegrenzungselements der zweiten Ausführungsform der Dosiervorrichtung der vorliegenden Erfindung;
- Figur 3a: einen Ausschnitt einer dritten Ausführungsform der Dosiervorrichtung der vorliegenden Erfindung;
- Figur 3b: eine Frontansicht der dritten Ausführungsform der Dosiervorrichtung der vorliegenden Erfindung
- Figur 3c: eine Seitenansicht der Gewindestange der dritten Ausführungsform der Dosiervorrichtung der vorliegenden Erfindung;
- Figur 3d: eine weitere Ansicht der Gewindestange der dritten Ausführungsform der Dosiervorrichtung der vorliegenden Erfindung;
- Figur 3e: eine Ansicht eines Drehbegrenzungselements der dritten Ausführungsform der Dosiervorrichtung der vorliegenden Erfindung;
- Figur 3f: eine weitere Ansicht des Drehbegrenzungselements der dritten Ausführungsform der Dosiervorrichtung der vorliegenden Erfindung;
- Figur 4a: einen Ausschnitt einer vierten Ausführungsform der Dosiervorrichtung der vorliegenden Erfindung;
- Figur 4b: den Ausschnitt der vierten Ausführungsform der Dosiervorrichtung der vorliegenden Erfindung aus Figur 4a mit geöffnetem Drehbegrenzungselement;
- Figur 4c: den Ausschnitt der vierten Ausführungsform der Dosiervorrichtung der vorliegenden Erfindung aus Figur 4a mit geöffnetem Drehbegrenzungselement;
- Figur 4d: eine Ansicht der Gewindestange der vierten Ausführungsform der Dosiervorrichtung der vorliegenden Erfindung;
- Figur 4e: eine Ansicht eines geöffneten Drehbegrenzungselements der vierten Ausführungsform der Dosiervorrichtung der vorliegenden Erfindung;
- Figur 4f: eine weitere Ansicht des Drehbegrenzungselements der vierten Ausführungsform der Dosiervorrichtung der vorliegenden Erfindung;
- Figur 5a: eine Injektionsvorrichtung mit einer Ausführungsform einer erfindungsgemäßen Dosiervorrichtung mit einer vollständig in die Injektionsvorrichtung eingedrehten Drehhülse;
- Figur 5b: einen Querschnitt der Injektionsvorrichtung aus Figur 5a; und
- Figur 5c: einen Querschnitt der Injektionsvorrichtung aus Figur 5a mit einer aufgezogenen Drehhülse.

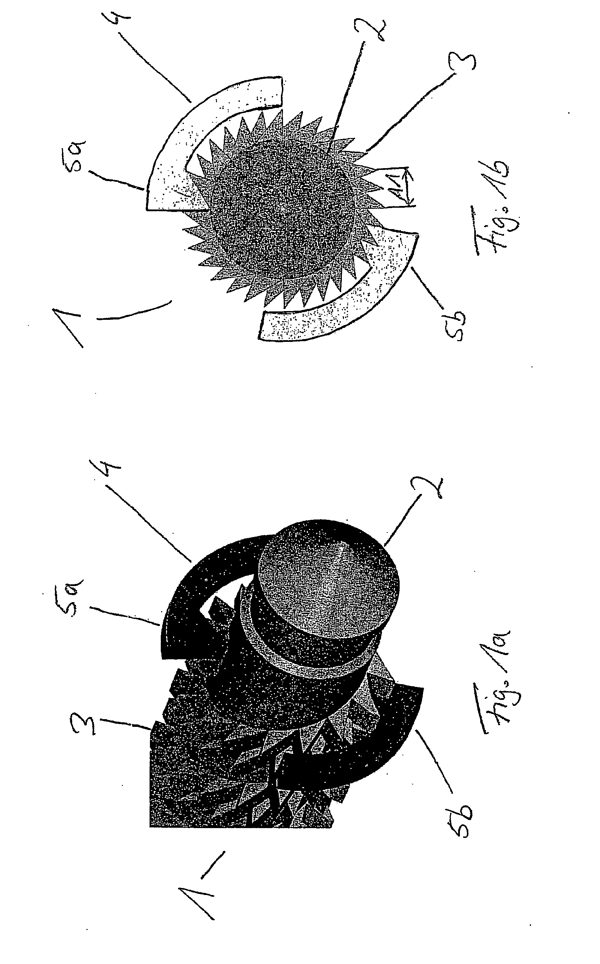

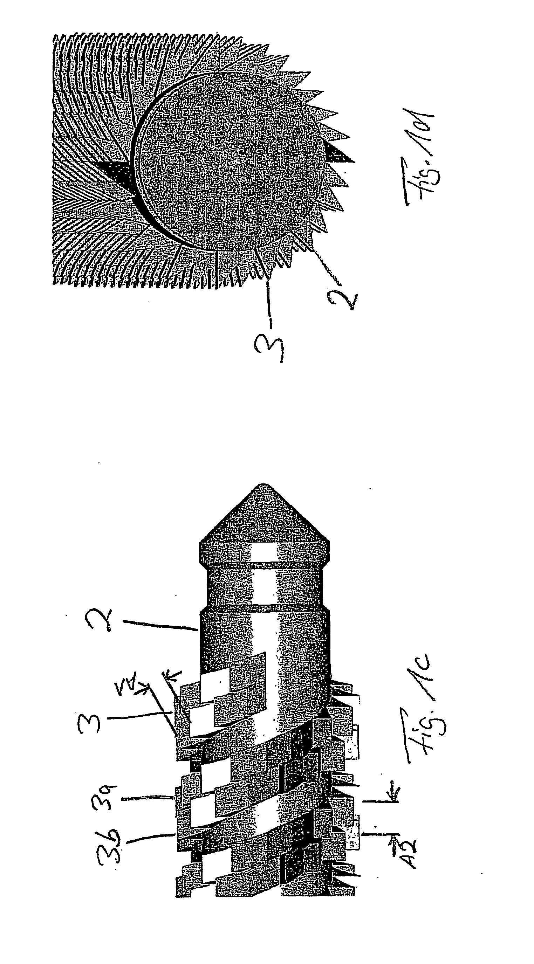

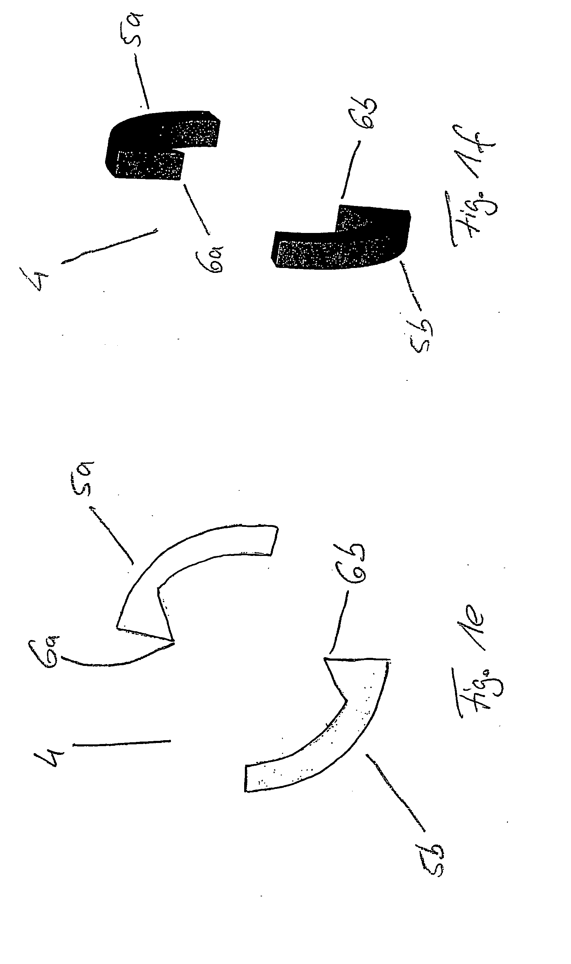

Die Figuren 1a bis 1f zeigen Ausschnitte und Teile einer Dosiervorrichtung 1 mit einer Gewindestange 2 gemäß einer ersten Ausführungsform der vorliegenden Erfindung. Die Gewindestange 2 weist ein Gewinde auf, welches in Umfangsrichtung der Gewindestange 2 eine Zahnung 3 aufweist, wobei jeweils zwei Zähne der Zahnung 3, wie in Figur 1c zu sehen, in Längsrichtung aneinander angrenzen oder anliegen. Auch kann diese Ausführungsform so verstanden werden, dass zwei aneinander angrenzende oder aneinander anliegende Zahnungen 3a, 3b von einem oder mehreren Gewinden getragen werden und die Gewindestange 2 umlaufen. Dabei sind die in Längsrichtung der Gewindestange 2 benachbarten Zähne der Zahnung 3 oder die in Längsrichtung aneinander angrenzenden Zahnungen 3a, 3b versetzt zueinander angeordnet. Die in Längsrichtung der Gewindestange 2 aneinander angrenzenden Zähne weisen in Längsrichtung einen zweiten Abstand A2 voneinander auf, wobei die Umfangsrichtung benachbarten Zähne der Zahnung 3 einen ersten Abstand A1 voneinander aufweisen. Um die Gewindestange 2 liegt ein mit einer Injektionsvorrichtung oder einem Gehäuseteil der Injektionsvorrichtung verbundenes Drehbegrenzungselement 4, welches zwei Rastelemente 5a, 5b aufweist, welche in Umfangsrichtung der Gewindestange 2 versetzt zueinander angeordnet sind. Ebenso sind die Rastelemente 5a, 5b in Längsrichtung der Gewindestange 2, wie in Figur 1f zu erkennen, versetzt zueinander angeordnet, wobei dieser Versatz dem zweiten Abstand A2 der in Längsrichtung benachbarten Zähne oder in Längsrichtung angrenzenden Zähne der Zahnung 3 entspricht, so dass die Zahnung 3 stets entlang der Rastelemente 5a, 5b geführt werden kann. In Umfangsrichtung der Gewindestange 2 liegen die Rastelemente 5a, 5b sich nicht genau gegenüber, der Abstand entlang des Umfangs der Gewindestange 2 zwischen den Eingriffsspitzen 6a, 6b der Rastelemente 5a, 5b entspricht also nicht genau dem halben Umfang der Gewindestange 2 oder die gedachten Verlängerungen der Eingriffsspitzen 6a, 6b schneiden sich nicht in dem Mittelpunkt oder in der Längsachse der Gewindestange 2. Der Unterschied oder die Differenz zu dem genauen halben Umfang als Abstand zwischen den Rastelementen 5a, 5b wird durch einen Versatz zwischen den Rastelementen 5a, 5b beschrieben. Der Versatz zwischen den Rastelementen 5a, 5b beträgt genau die Hälfte des ersten Abstands A1 in Umfangsrichtung zwischen zwei in Längsrichtung an gleicher Stelle angeordneten Zähnen der Zahnung 3, so dass ein Rastelement 5a mit der Zahnung 3 verrastet ist, während das andere Rastelement 5b, wie in Figur 1b zu sehen, nicht mit der Zahnung 3 verrastet ist. Werden die Rastelemente 5a, 5b, zum Beispiel zum Einstellen einer Dosis, relativ zu der Gewindestange 2 um einen Betrag oder einen Weg kleiner als der erste Abstand A1 zwischen zwei benachbarten Zähnen gedreht, so wird die Verrastung des ersten Rastelements 5a mit der Zahnung 3 gelöst, das erste Rastelement 5a rastet jedoch nicht erneut mit einem benachbarten, durch den ersten Abstand A1 beabstandeten Zahn ein, sondern das zweite Rastelement 5b rastet in einer Zwischenposition mit der Zahnung 3 ein, indem das zweite Rastelement 5b mit einem zu seiner Ausgangsposition benachbarten Zahn verrastet. Durch eine erneute Drehung wird die Verrastung des zweiten Rastelements 5b mit der Zahnung 3 gelöst und das erste Rastelement 5a kann schon nach einem Weg, welcher kleiner ist als der erste Abstand A1, mit einem zu seiner vorherigen Position benachbarten Zahn verrasten. Somit können kleinere Dosen eingestellt werden, als bei Dosiervorrichtungen 1, welche nur die Bewegung eines Rastelements 5a, 5b zum jeweils in Umfangsrichtung benachbarten Zahn als kleinste Dosiseinstellung ermöglichen.

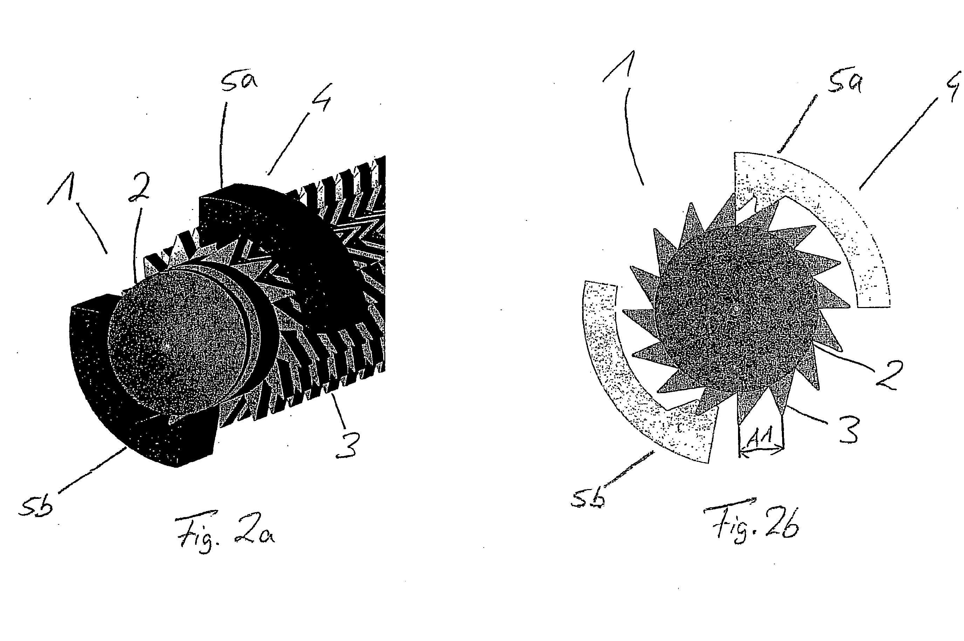

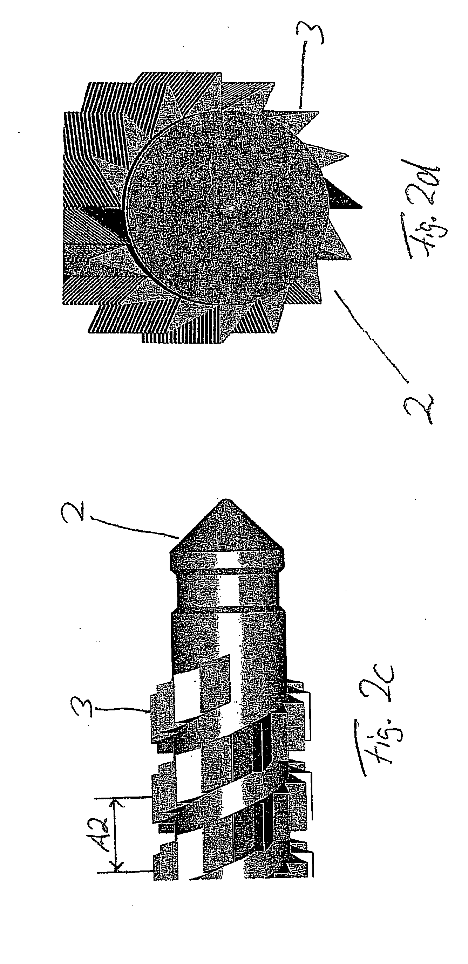

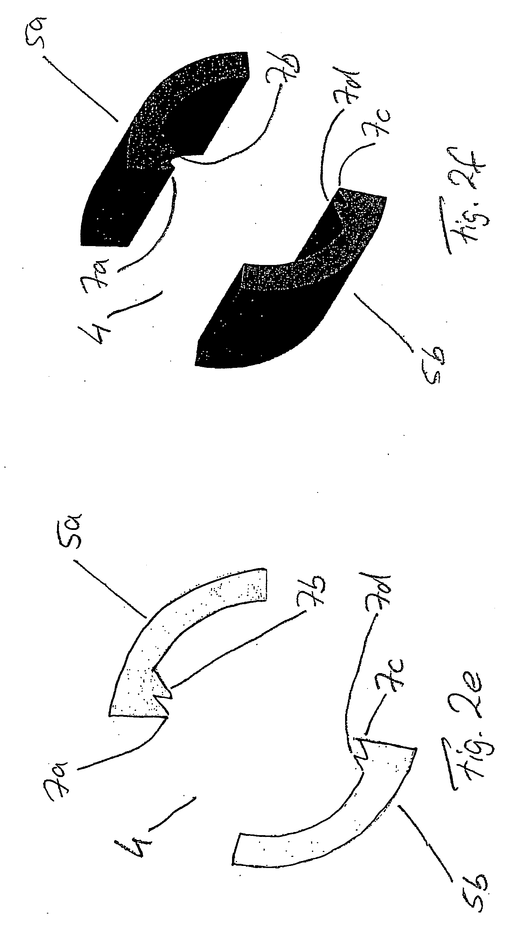

Die Figuren 2a bis 2f zeigen Ausschnitte und Teile einer Dosiervorrichtung 1 mit einer Gewindestange 2 gemäß einer zweiten Ausführungsform der vorliegenden Erfindung mit einer entlang eines Gewindes die Gewindestange 2 gleichmäßig umlaufenden Zahnung 3. Die Zähne der Gewindestange 2 weisen in Umfangsrichtung einen ersten konstanten Abstand A1 voneinander auf, sowie in Längsrichtung einen zweiten konstanten Abstand A2 voneinander auf. Die in Längsrichtung benachbarten Zähne sind weiter in Umfangsrichtung der Gewindestange 2 an gleicher Position angeordnet oder nicht versetzt zueinander angeordnet. Eine feinere Dosierung wird in dieser Ausführungsform unter Anderem wie bei der ersten Ausführungsform durch einen Versatz der Rastelemente 5a, 5b zueinander, verglichen mit einem idealen Gegenüberliegen, erreicht, was in Figur 2e zu erkennen ist. Auch hier beträgt der Versatz die Hälfte des ersten Abstands A1 der Zähne in Umfangsrichtung voneinander. Weiter wird eine feinere Einstellung einer Dosis dadurch erreicht, dass an jedem der Rastelemente 5a, 5b zwei Eingriffselemente 7a, 7b ausgebildet sind, deren Abstand voneinander der Hälfte des Abstands A1 entspricht. Wird das Drehbegrezungselement 4 aus einer Grundposition, wie in Figur 2b gezeigt, relativ zu der Gewindestange 2 um einen Betrag gegen den Uhrzeigersinn gedreht, der kleiner als der Abstand A1 der Zähne in Umfangsrichtung zueinander ist, so verrastet zumindest eines der Rastelemente 5a, 5b mit dem selben oder darauf folgenden oder benachbarten Zahn schon nach einem Weg, der der Hälfte des Abstands A1 entspricht, so dass eine halb so große Dosis eingestellt werden kann, wie mit anderen Dosiervorrichtungen 1, welche den selben Abstand A1 der Zähne in Umfangsrichtung voneinander aufweisen.

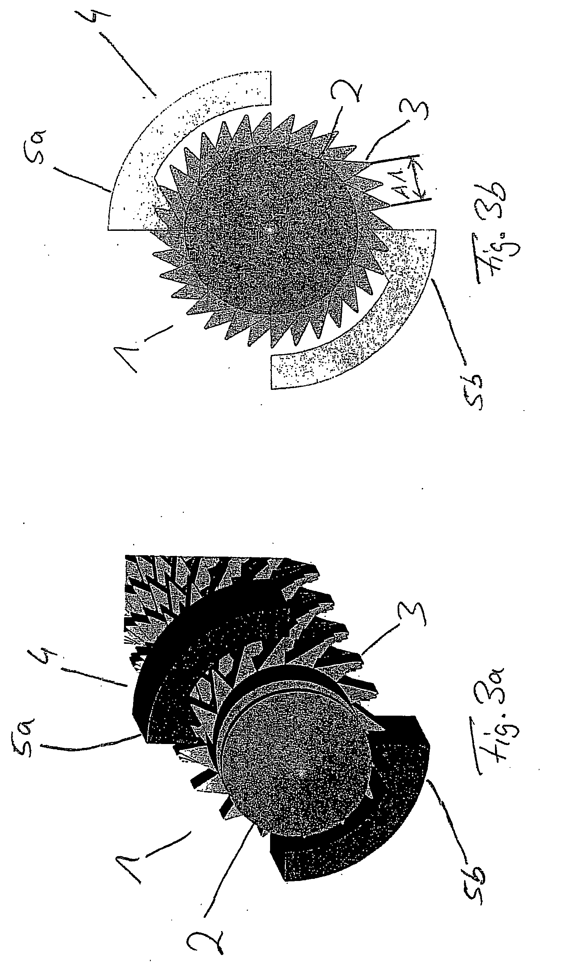

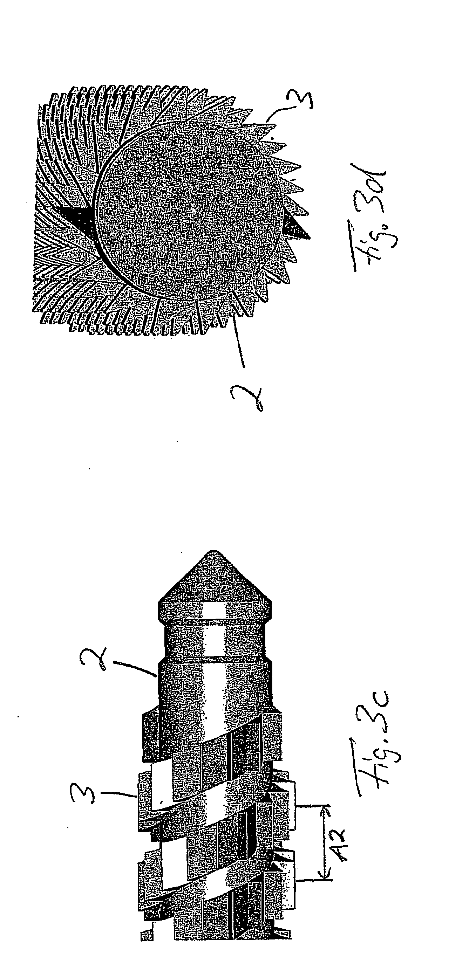

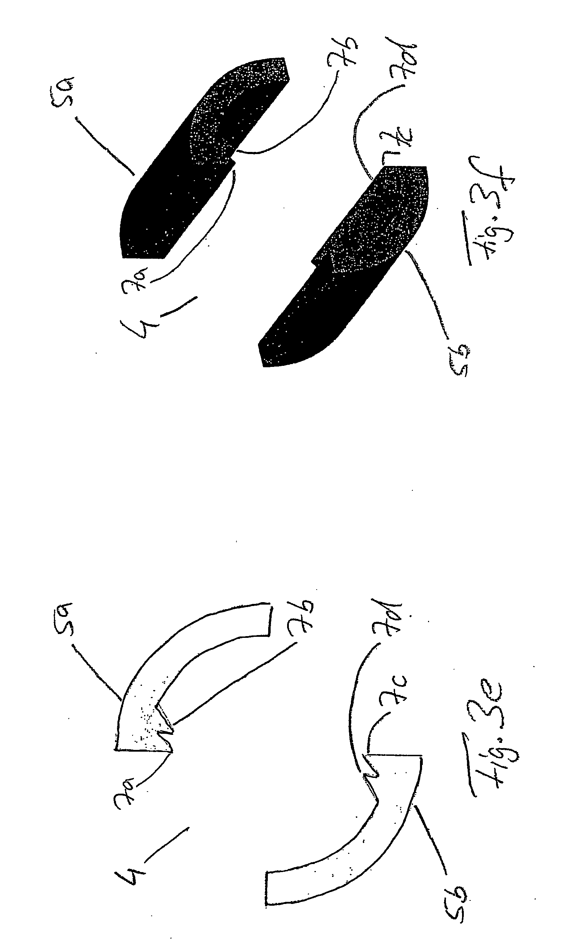

Die Figuren 3a bis 3f zeigen Ausschnitte und Teile einer Dosiervorrichtung 1 mit einer Gewindestange 2 gemäß einer dritten Ausführungsform der vorliegenden Erfindung mit einer in Umfangsrichtung der Gewindestange 2 ausgebildeten Zahnung 3 entlang eines Gewindes auf der Gewindestange 2, wobei die benachbarten Zähne in Umfangsrichtung der Zahnstange 2 einen konstanten ersten Abstand A1 voneinander aufweisen und die in Längsrichtung benachbarten Zähne, wie die schräg benachbarten Zähne, in Längsrichtung der Gewindestangen 2 einen zweiten konstanten Abstand A2 voneinander aufweisen. Die feinere Dosierung wird in dieser Ausführungsform unter Anderem dadurch erreicht, dass jeweils zwei Zähne in Längsrichtung der Gewindestange 2 versetzt zueinander angeordnet sind, in Umfangsrichtung der Gewindestange 2 also nicht die gleiche Position aufweisen. Weiter weisen die beiden Rastelemente 5a, 5b zwei Eingriffselemente 7a, 7b auf, welche eine Einstellung einer feineren Dosismenge gewährleisten. Ist zum Beispiel, wie in Figur 3b gezeigt, eines der Rastelemente 5a, 5b mit der Zahnung 3 verrastet, liegt beispielsweise die senkrecht zu der Gewindestange 2 stehende Anschlagsflanke des Rastelements 5a an einer senkrecht zu der Gewindestange 2 stehenden Gegenanschlagsflanke des Zahns oder Nockens an, so kann die Gewindestange 2 relativ zu den Rastelementen 5a, 5b nicht im Uhrzeigersinn gedreht werden oder die Rastelemente 5a, 5b können nicht relativ zu der Gewindestange entgegen des Uhrzeigersinns gedreht werden, da diese Bewegungsrichtung zum Beispiel durch das mit der Zahnung 3 verrastete, Rastelement 5a blockiert wird, sondern die Gewindestange 2 kann relativ zu den Rastelementen 5a, 5b nur gegen den Uhrzeigersinn gedreht werden oder die Rastelemente 5a, 5b können relativ zu der Gewindestange 2 nur im Uhrzeigersinn gedreht werden. Werden die Rastelemente 5a, 5b, beispielsweise zum Einstellen einer Dosis, im Uhrzeigersinn relativ zu der Gewindestange 2 um einen kleineren Betrag als den Abstand A1 zwischen zwei in Umfangsrichtung benachbarten Zähnen gedreht, so rastet zumindest eines der Rastelemente 5a, 5b wieder mit der Verzahnung in einer Zwischenposition ein bevor es ein dem Abstand A1 entsprechenden Weg zurückgelegt hat. Der zurückgelegte Weg entspricht in der vorliegenden Ausführungsform der Hälfte des ersten Abstands A1, kann jedoch auch je nach Anordnung der Zahnung 3 und der Rastelemente 5a, 5b einem Drittel, einem Viertel oder einem anderen Teil des ersten Abstands A1 entsprechen. In der Zwischenposition liegt ein Zahn der Zahnung 3 zwischen den zwei Eingriffselementen mindestens eines der Rastelemente 5a, 5b oder eine Anschlagsflanke mindestens eines der Rastelemente 5a, 5b liegt an einem Gegenanschlag eines Zahns an. Da zum Einstellen einer kleinstmöglichen Dosis nur eine dem halben Abstand A1 zwischen zwei Zähnen entsprechende Drehung der Rastelemente 5a, 5b relativ zu der Gewindestange 2 bis zu einer erneuten Verrastung oder bis zu einem erneuten Einrasten gedreht werden kann oder gedreht wird, kann eine doppelt so feine Dosierung oder kann eine halb so große Dosismenge mit der Dosiervorrichtung 1 eingestellt und abgegeben werden.

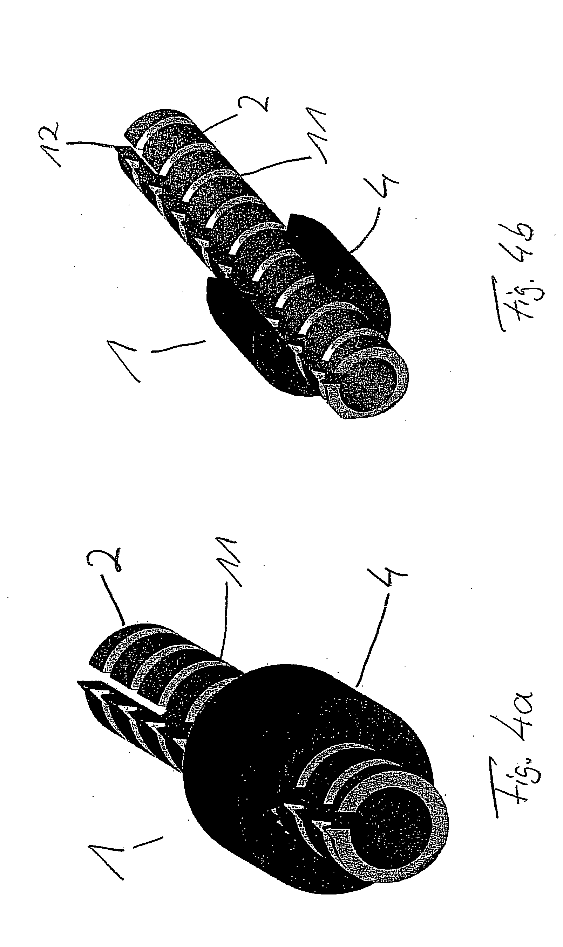

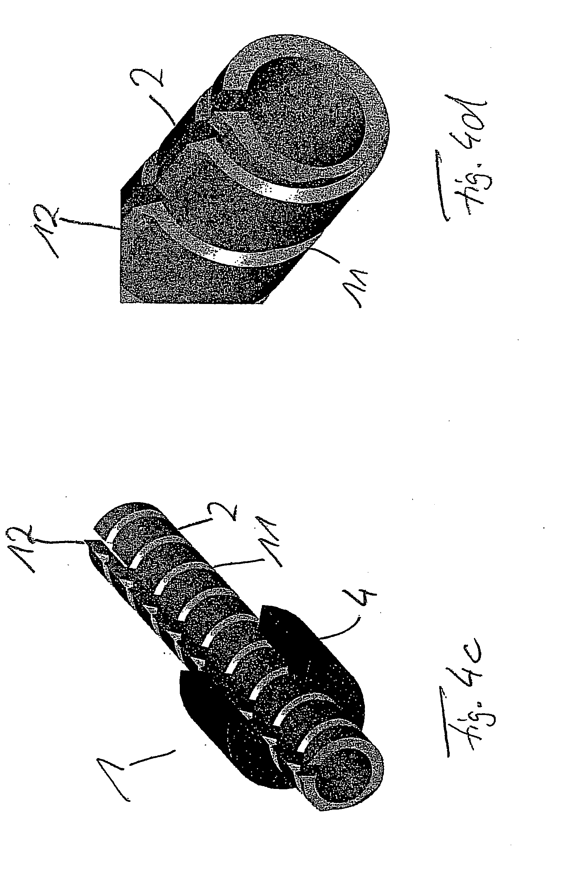

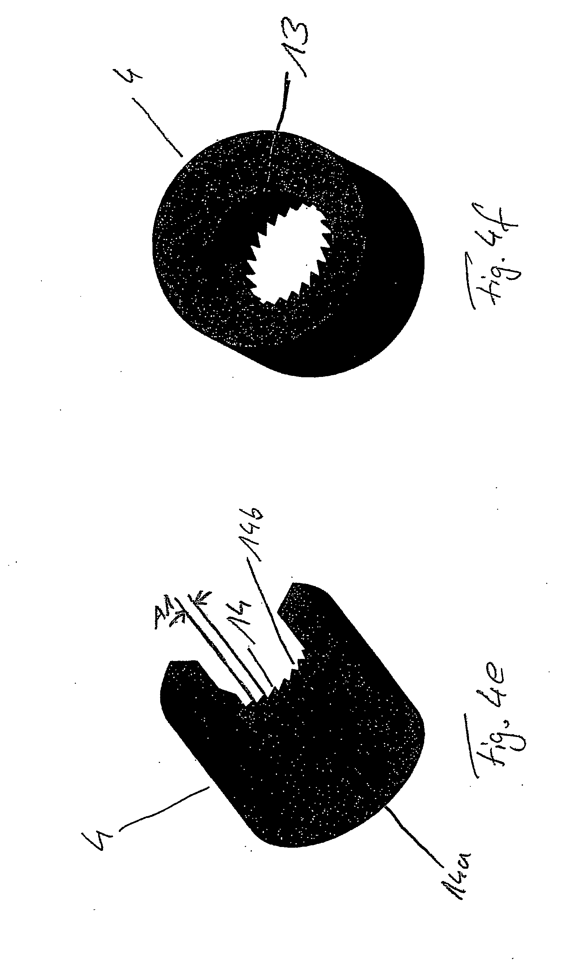

Die Figuren 4a bis 4f zeigen Ausschnitte und Teile einer Dosiervorrichtung gemäß einer vierten Ausführungsform der vorliegenden Erfindung mit einer Gewindestange 2, welche eine umlaufende gewindeförmige Führungsrille 11 und eine Vielzahl von Eingriffselementen 12 aufweist. Die Eingriffselemente 12 sind entlang einer Parallelen zur Längsachse der Gewindestange 2 ausgebildet. Weiter umfasst die Dosiervorrichtung 1 ein Drehbegrenzungselement 4, welches zum Beispiel mit der Injektionsvorrichtung oder mit einer Drehhülse der Injektionsvorrichtung verbunden sein kann. Das Drehbegrenzungselement 4 weist auf der Innenseite eine Zahnung 14 und ein Führungselement 13 auf. Das Führungselement 13 ist so ausgebildet, dass es in die Führungsrille 11 der Gewindestange eingreifen kann und entlang der Gewindestange 2 geführt und gedreht werden kann, so dass das Drehbegrenzungselement 4 eine relative Drehbewegung und eine relative translatorische Bewegung bezüglich der Gewindestange 3 ausführen kann. Die Zahnung 14 des Drehbegrenzungselements 4 ist so ausgebildet, dass sie eine erste Teilzahnung 14a und eine zweite Teilzahnung 14b aufweist, welche in Längsrichtung des Drehbegrenzungseleinents versetzt zu der ersten Teilzahnung 14a ausgebildet ist. Die Abstände der benachbarten Zähne jeder Teilzahnung 14a, 14b in Umfangsrichtung voneinander entsprechen einem ersten Abstand A1. Die in Längsrichtung des Drehbegrenzungselements 4 benachbarten oder schräg benachbarten Zähne der Teilzahnungen 14a, 14b sind in Umfangsrichtung des Drehbegrenzungselements 4 versetzt zueinander angeordnet, wobei der Versatz der Hälfte des ersten Abstands A1 zwischen zwei in Umfangsrichtung benachbarten Zähnen einer Teilzahnung 14a, 14b entspricht. Der Versatz kann auch einem Drittel, einem Viertel oder einem anderen beliebigen Teil des ersten Abstands A1 entsprechen. Durch diesen Versatz können abwechselnd die beiden Teilzahnungen 14a, 14b mit einem der Eingriffselemente 12 der Gewindestange verrasten. Aufgrund des abwechselnden Verrastens können feinere Drehbewegungen bis zu einem Verrasten oder Einrasten ausgeführt werden, als bei einem Verrasten in Umfangsrichtung benachbarter Zähne einer Teilzahnung 14a, 14b mit einem der Eingriffselemente 12. Da die in Längsrichtung benachbarten Zähne der Teilzahnungen 14a, 14b in Umfangsrichtung den halben ersten Abstand A1 voneinander aufweisen, können doppelt so feine Dosierungen mit der Dosiervorrichtung eingestellt werden wie bei dem Gebrauch eines herkömmlichen Drehbegrenzungselements.

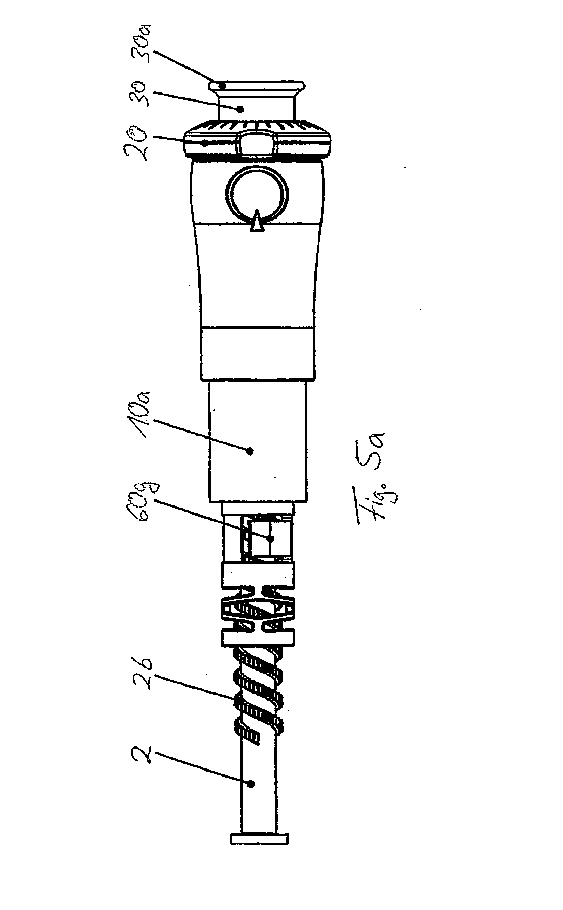

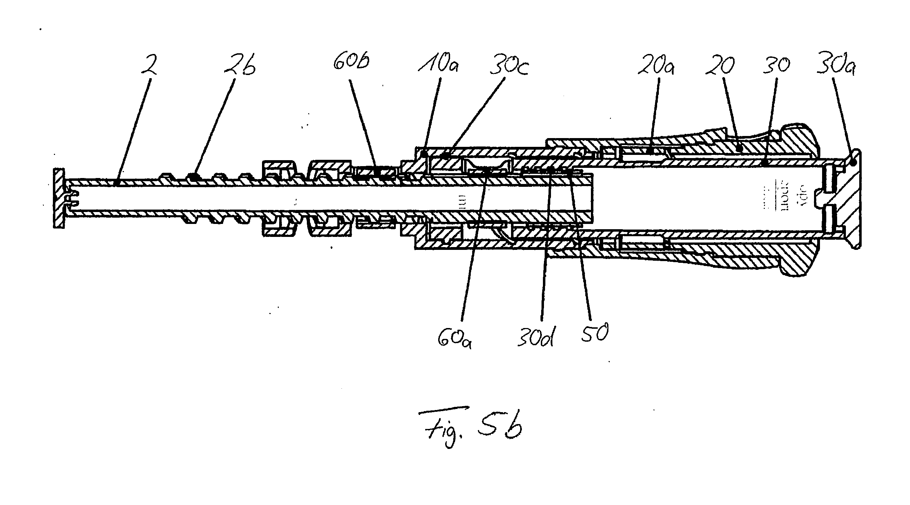

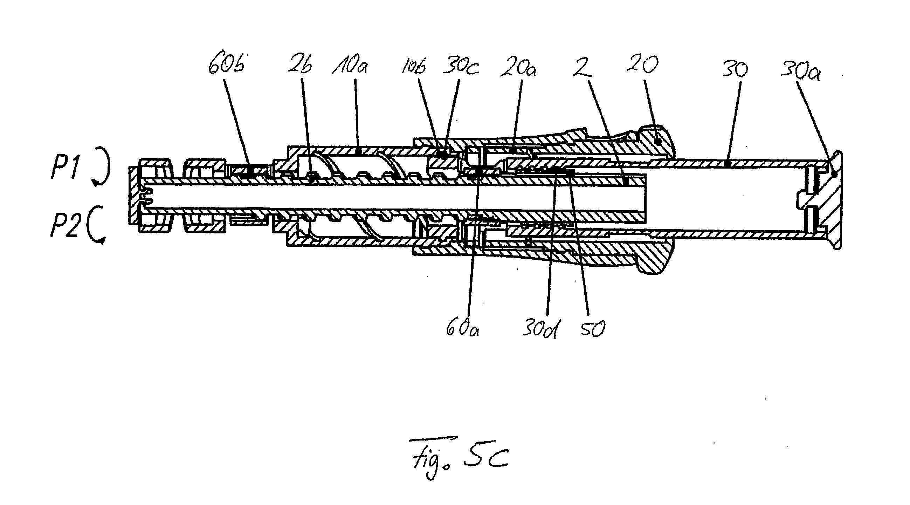

Die Figuren 5a, 5b und 5c zeigen eine Injektionsvorrichtung mit einer Ausführungsform einer erfindungsgemäßen Dosiervorrichtung. In den Figuren 5a und 5b ist eine Drehhülse 30 vollständig in die Injektionsvorrichtung eingedreht, wobei die Drehhülse 30 über ein Außengewinde 30c in einem Innengewinde 10b des Injektionsvorrichtungsgehäuses 10a geführt ist und ein Innengewinde 30d aufweist. Wird die Injektionsvorrichtung durch ein Herausdrehen der Drehhülse 30 aufgezogen, wie in Figur 5c gezeigt, so wird die Gewindestange 2, welche ein verdrehgesichert aber axial verschiebbar auf der Gewindestange 2 gelagertes Sperrelement 50 aufweist, von einem mit der Injektionsvorrichtung oder einem Gehäuseteil 10a der Injektionsvorrichtung verbundenen Drehbegrenzungselement 60b verdrehgesichert relativ zum Injektionsvorrichtungsgehäuse 10a gehalten, während ein mit der Drehhülse 30 verbundenes Drehbegrenzungselement 60a bei der in der durch den Pfeil P1 angegebenen Drehrichtung, welche ein Herausdrehen der Drehhülse 30 aus dem Gehäuse 10a bewirkt, nicht in Blockiereingriff mit der Zahnung auf dem Gewindegang 2b der Gewindestange 2 ist. Das Drehbegrenzungselement 60a weist Rastelemente auf, welche an elastischen um die Gewindestange 2 umlaufenden Armen 60g angebracht sind. Beim Wiedereindrehen der drehbar in einem Drehknopf 30a gelagerten Drehhülse 30 in der in Figur 5c gezeigten durch den Pfeil P2 gezeigten Richtung, z.B. durch Drücken des Drehknopfes 10a, sind die Eingriffselemente des Drehbegrenzungselements 60a der Drehhülse 30 im Eingriff mit der Zahnung des Gewindes 2a der Gewindestange 2, wodurch während des Eindrehens der Drehhülse 30 die Gewindestange 2 drehsicher mit der Drehhülse 30 gekoppelt ist und von dieser mitgenommen und mitgedreht wird. Die Drehhülse 30 kann soweit eingedreht werden bis ein vorderes oder distales Ende einer Einstellhülse 20, welches einen Einstellsperrnocken 20a aufweist, an einem Innenanschlag der Injektionsvorrichtung anliegt. Das Drehbegrenzungselement 60b der Injektionsvorrichtung ermöglicht eine Drehung der Gewindestange 2 relativ zu der Injektionsvorrichtung bzw. dem Gehäuse 10a, so dass die Gewindestange 2 durch die Drehhülse 30 gedreht und in dem Innengewinde der Injektionsvorrichtung bzw. dem Gehäuse 10a geführt wird und sich so aus dem Gehäuse 10a in distale Richtung herausdreht, um eine eingestellte und aufgezogene Dosis abzugeben. Anschließend kann die Injektionsvorrichtung durch ein Herausziehen der Drehhülse 30 in der durch den Pfeil P1 in Figur 5c angegebenen Richtung aufgezogen werden.

## Patentansprüche

1. Dosiervorrichtung (1) zur dosierten Verabreichung eines injizierbaren Produkts aus einer Injektionsvorrichtung mit:
- einer Gewindestange (2) mit einem umlaufenden in Umfangsrichtung eine Zahnung (3) tragenden Gewinde, wobei entlang des Gewindegangs des Gewindes die in Umfangsrichtung der Gewindestange (2) benachbarten Zähne der Zahnung einen ersten Abstand (A1) in Umfangsrichtung voneinander aufweisen;
- einem mit der Injektionsvorrichtung oder einem Gehäuseteil der Injektionsvorrichtung verbundenen ersten Drehbegrenzungselement (4) mit mindestens einem Rastelement (5a, 5b), insbesondere einem, zwei, drei oder vier Rastelementen (5a, 5b), zum Verrasten mit der Zahnung (3) der Gewindestange (2);
- einem mit einer Drehhülse der Injektionsvorrichtung verbundenen zweiten Drehbegrenzungselement (4) mit mindestens einem Rastelement (5a, 5b), insbesondere zwei, drei oder vier Rastelementen (5a, 5b), zum Verrasten mit der Zahnung (3) der Gewindestange (2);
wobei
das mindestens eine Rastelement (5a, 5b) der Drehbegrenzungselemente (4) so angeordnet oder ausgebildet ist, dass bei einer Drehung der Gewindestange (2) relativ zu mindestens einem der Drehbegrenzungselemente (4) um weniger als den ersten Abstand (A1) das mindestens eine Rastelement (5a, 5b) in einer Zwischenposition verrasten kann.

2. Dosiervorrichtung (1) nach Anspruch 1 , wobei die in Längsrichtung der Gewindestange (2) benachbarten Zähne in Umfangsrichtung, insbesondere um einen der Hälfte des ersten Abstandes (A1) entsprechenden Abstand, versetzt zueinander angeordnet sind.

3. Dosiervorrichtung (1) nach einem der vorhergehenden Ansprüche, wobei mindestens ein Drehbegrenzungselement (4) n Rastelemente (5a, 5b) aufweist und die in Längsrichtung der Gewindestange (2) benachbarten Zähne in Umfangsrichtung um den n-ten Teil des ersten Abstands (A1) versetzt zueinander angeordnet sind.

4. Dosiervorrichtung (1) nach Anspruch 1, wobei die in Längsrichtung der Gewindestange (2) benachbarten Zähne in Umfangsrichtung an gleicher Position angeordnet sind.

5. Dosiervorrichtung (1) nach einem der vorhergehenden Ansprüche, wobei mindestens eines der Drehbegrenzungselemente (4) zwei Rastelemente (5a, 5b) aufweist und die Eingriffsspitzen (6a, 6b) der Rastelemente (5a, 5b) in Umfangsrichtung der Gewindestange voneinander beabstandet sind, wobei der Abstand um einen vorgegebenen Versatz von einem ganzzahligen Vielfachen des ersten Abstands (A1) abweicht.

6. Dosiervorrichtung (1) nach einem der vorhergehenden Ansprüche, wobei mindestens eines der Drehbegrenzungselemente (4) n Rastelemente (5a, 5b) aufweist und die Eingriffsspitzen (6a, 6b) der Rastelemente (5a, 5b) in Umfangsrichtung der Gewindestange voneinander beabstandet sind, wobei der Abstand um einen vorgegebenen Versatz von einem ganzzahligen Vielfachen des ersten Abstands (A1) abweicht und der Versatz dem n-ten Teil des ersten Abstands (A1) entspricht.

7. Dosiervorrichtung (1) nach einem der vorhergehenden Ansprüche, wobei das mindestens eine Rastelement (5a, 5b) mindestens zwei Eingriffselemente (7a, 7b), insbesondere zwei, drei, oder vier, aufweist, wobei die Eingriffselemente (7a, 7b) jeweils einen konstanten Eingriffselementabstand voneinander aufweisen.

8. Dosiervorrichtung (1) nach einem der vorhergehenden Ansprüche, wobei das mindestens eine Rastelement (5a, 5b) n Eingriffselemente (7a, 7b) aufweist, wobei die Eingriffselemente (7a, 7b) jeweils einen konstanten Eingriffselementabstand voneinander aufweisen und der Eingriffselementabstand dem n-ten Teil des ersten Abstands (A1) entspricht.

9. Dosiervorrichtung (1) nach einem der vorhergehenden Ansprüche, wobei mindestens zwei der in Längsrichtung der Gewindestange (2) benachbarten Zähne der Zahnung (3) einen zweiten Abstand (A2) in Längsrichtung voneinander aufweisen.

10. Dosiervorrichtung (1) nach dem vorhergehenden Anspruch, wobei mindestens eines der Drehbegrenzungselemente (4) mindestens zwei Rastelemente (5a, 5b) aufweist und die Rastelemente (5a, 5b) in Längsrichtung der Gewindestange (2) um den zweiten Abstand (A2) beabstandet sind.

11. Dosiervorrichtung (1) nach einem der vorhergehenden Ansprüche, wobei die Rastelemente (5a, 5b) so ausgebildet sind, dass sie sich in Längsrichtung der Gewindestange (2) entlang mindestens zwei Zähnen, insbesondere entlang zwei, drei, vier oder n Zähnen, der Zahnung (3) der Gewindestange (2) erstrecken.

12. Dosiervorrichtung (1) nach einem der vorhergehenden Ansprüche, wobei der Versatz zwischen den Rastelementen (5a, 5b) so ausgebildet ist, dass in einem ersten Zustand der Dosiervorrichtung (1) die Anschlagsflanke eines ersten Rastelements (5a, 5b) an einem Gegenanschlag eines Zahns der Zahnung (3) anliegt, in welchem die Anschlagsflanke eines zweiten Rastelements (5a, 5b) nicht an einem Gegenanschlag eines Zahns der Zahnung (3) anliegt und in einem zweiten Zustand die Anschlagsflanke des zweiten Rastelements (5a, 5b) an einem Gegenanschlag eines Zahns der Zahnung (3) anliegt, in welchem die Anschlagsflanke des ersten Rastelements (5a, 5b) nicht an einem Gegenanschlag eines Zahns der Zahnung (3) anliegt

13. Dosiervorrichtung (1) nach einem der vorhergehenden Ansprüche, wobei der zweite Abstand (A2) kleiner ist als der erste Abstand (A1) oder der erste Abstand (A1) kleiner ist als der zweite Abstand (A2).

14. Dosiervorrichtung (1) nach einem der vorhergehenden Ansprüche, wobei die Drehbegrenzungselemente (4) relativ zu der Gewindestange (2) radial bewegbar sind.

15. Dosiervorrichtung (1) nach einem der vorhergehenden Ansprüche, wobei die Zähne der Zahnung (3) in einem ersten entlang der Längsachse der Gewindestange (2) ausgebildeten Abschnitt jeweils einen konstanten Abstand in Umfangsrichtung der Gewindestange (2) voneinander aufweisen und in einem benachbarten zweiten entlang der Längsachse der Gewindestange (2) ausgebildeten Abschnitt jeweils einen weiteren konstanten Abstand in Umfangsrichtung der Gewindestange (2) voneinander aufweisen, wobei der Abstand der Zähne in dem ersten Abschnitt und der Abstand der Zähne in dem zweiten Abschnitt unterschiedlich sind.

16. Dosiervorrichtung (1) zur dosierten Verabreichung eines injizierbaren Produkts aus einer Injektionsvorrichtung mit:
- einer Gewindestange (2) mit einer umlaufenden gewindeförmigen Führungsrille (11) und einer Vielzahl von, insbesondere entlang einer Parallelen zur Längsachse der Gewindestange (2) ausgebildeten, Eingriffselementen (12);
- einem mit der Injektionsvorrichtung oder einem Gehäuseteil der Injektionsvorrichtung verbundenen und die Gewindestange (2), insbesondere vollständig, umgebenden ersten Drehbegrenzungselement (4) mit einem auf der Innenseite des Drehbegrenzungselements (4) ausgebildeten Führungselement (13) zum Eingreifen in die Führungsrille (11) und einer auf der Innenseite des Drehbegrenzungselements (4) ausgebildeten Zahnung (14) zum Verrasten mit den Eingriffselementen (12), wobei die in Längsrichtung des ersten Drehbegrenzungselements (4) an gleicher Position angeordneten in Umfangsrichtung des Drehbegrenzungselements (4) benachbarten Zähne der Zahnung (14) einen ersten Abstand (A1) in Umfangsrichtung voneinander aufweisen;
- einem mit einer Drehhülse der Injektionsvorrichtung verbundenen und die Gewindestange (2), insbesondere vollständig, umgebenden zweiten Drehbegrenzungselement (4) mit einem auf der Innenseite des Drehbegrenzungselements (4) ausgebildeten Führungselement (13) zum Eingreifen in die Führungsrille (11) und einer auf der Innenseite des Drehbegrenzungselements (4) ausgebildeten Zahnung (14) zum Verrasten mit den Eingriffselementen (12), wobei die in Längsrichtung des zweiten Drehbegrenzungselements (4) an gleicher Position angeordneten in Umfangsrichtung des Drehbegrenzungselements (4) benachbarten Zähne der Zahnung (14) einen ersten Abstand (A1) in Umfangsrichtung voneinander aufweisen;
wobei
die Zahnung (14) der Drehbegrenzungselemente (4) so angeordnet oder ausgebildet ist, dass bei einer Drehung der Gewindestange (2) relativ zu mindestens einem der Drehbegrenzungselemente (4) um weniger als den ersten Abstand (A1) mindestens eines der Eingriffselemente (12) in einer Zwischenposition mit der Zahnung (14) verrasten kann.

17. Dosiervorrichtung (1) nach dem vorhergehenden Anspruch, wobei die Zahnung (14) aus mindestens zwei, insbesondere aus zwei, drei, vier, fünf oder sechs, in Längsrichtung des Drehbegrenzungselements (4) versetzt zueinander angeordneten Teilzahnungen (14a, 14b) besteht, wobei die in Längsrichtung des Drehbegrenzungselements (4) benachbarten Zähne der benachbarten Teilzahnungen (14a, 14b) in Umfangsrichtung des Drehbegrenzungselements (4), insbesondere um einen der Hälfte oder einem Drittel oder einem Viertel oder einem Fünftel oder einem Sechstel des ersten Abstands (A1) entsprechenden Abstand, versetzt zueinander angeordnet sind.

18. Dosiervorrichtung (1) nach einem der zwei vorhergehenden Ansprüche, wobei die Zahnung (14) über die gesamte Länge der Drehbegrenzungselemente (14) ausgebildet ist.

19. Dosiervorrichtung (1) nach einem der drei vorhergehenden Ansprüche, wobei die Zähne der Zahnung (14) in einem ersten entlang der Längsachse des Drehbegrenzungselements (4) ausgebildeten Abschnitt jeweils einen konstanten Abstand in Umfangsrichtung des Drehbegrenzungselements (4) voneinander aufweisen und in einem benachbarten zweiten entlang der Längsachse des Drehbegrenzungselements (4) ausgebildeten Abschnitt jeweils einen anderen konstanten Abstand in Umfangsrichtung des Drehbegrenzungselements (4) voneinander aufweisen, wobei der Abstand der Zähne in dem ersten Abschnitt und der Abstand der Zähne in dem zweiten Abschnitt unterschiedlich sind.

20. Injektionsvorrichtung mit einer Dosiervorrichtung (1) nach einem der vorhergehenden Ansprüche.

## Claims

1. A dose metering device (1) for the metered administration of an injectable product from an injection device comprising:
- a screwthreaded rod (2) with a peripherally extending screwthread carrying a tooth arrangement (3) in the peripheral direction, wherein along the screwthread flight of the screwthread the teeth of the tooth arrangement, that are adjacent in the peripheral direction of the screwthreaded rod (2), are at a first spacing (A1) from each other in the peripheral direction;
- a first rotation-limiting element (4) which is connected to the injection device or a housing portion of the injection device and which has at least one latching element (5a, 5b), in particular one, two, three or four latching elements (5a, 5b), for latching engagement with the tooth arrangement (3) of the screwthreaded rod (2); and
- a second rotation-limiting element (4) which is connected to a rotary sleeve of the injection device and which has at least one latching element (5a, 5b), in particular two, three or four latching elements (5a, 5b), for latching engagement with the tooth arrangement (3) of the screwthreaded rod (2);
wherein the at least one latching element (5a, 5b) of the rotation-limiting elements (4) is of such a configuration or arrangement that in a rotary movement of the screwthreaded rod (2) relative to at least one of the rotation-limiting elements (4) by less than the first spacing (A1) the at least one latching element (5a, 5b) can latch in an intermediate position.

2. A dose metering device (1) according to claim 1 wherein the teeth which are adjacent in the longitudinal direction of the screwthreaded rod (2) are arranged in mutually displaced relationship in the peripheral direction, in particular by a spacing corresponding to half of the first spacing (A1).

3. A dose metering device (1) according to one of the preceding claims wherein at least one rotation-limiting element (4) has n latching elements (5a, 5b) and the teeth which are adjacent in the longitudinal direction of the screwthreaded rod (2) are arranged in mutually displaced relationship in the peripheral direction by the n-th part of the first spacing (A1).

4. A dose metering device (1) according to claim 1 wherein the teeth which are adjacent in the longitudinal direction of the screwthreaded rod (2) are arranged at the same position in the peripheral direction.

5. A dose metering device (1) according to one of the preceding claims wherein at least one of the rotation-limiting elements (4) has two latching elements (5a, 5b) and the engagement tips (6a, 6b) of the latching elements (5a, 5b) are spaced from each other in the peripheral direction of the screwthreaded rod, wherein the spacing differs by a predetermined displacement from an integral multiple of the first spacing (A1).

6. A dose metering device (1) according to one of the preceding claims wherein at least one of the rotation-limiting elements (4) has n latching elements (5a, 5b) and the engagement tips (6a, 6b) of the latching elements (5a, 5b) are spaced from each other in the peripheral direction of the screwthreaded rod, wherein the spacing differs by a predetermined displacement from an integral multiple of the first spacing (A1) and the displacement corresponds to the n-th part of the first spacing (A1).

7. A dose metering device (1) according to one of the preceding claims wherein the at least one latching element (5a, 5b) has at least two engagement elements (7a, 7b), in particular two, three or four, wherein the engagement elements (7a, 7b) are respectively at a constant engagement element spacing from each other.

8. A dose metering device (1) according to one of the preceding claims wherein the at least one latching element (5a, 5b) has n engagement elements (7a, 7b), wherein the engagement elements (7a, 7b) are respectively at a constant engagement element spacing from each other and the engagement element spacing corresponds to the n-th part of the first spacing (A1).

9. A dose metering device (1) according to one of the preceding claims wherein at least two of the teeth of the tooth arrangement (3), that are adjacent in the longitudinal direction of the screwthreaded rod (2), are at a second spacing (A2) from each other in the longitudinal direction.

10. A dose metering device (1) according to the preceding claim wherein at least one of the rotation-limiting elements (4) has at least two latching elements (5a, 5b) and the latching elements (5a, 5b) are spaced in the longitudinal direction of the screwthreaded rod (2) by the second spacing (A2).

11. A dose metering device (1) according to one of the preceding claims wherein the latching elements (5a, 5b) are of such a configuration that they extend in the longitudinal direction of the screwthreaded rod (2) along at least two teeth, in particular along two, three, four or n teeth, of the tooth arrangement (3) of the screwthreaded rod (2).

12. A dose metering device (1) according to one of the preceding claims wherein the displacement between the latching elements (5a, 5b) is of such a configuration that in a first state of the dose metering device (1) the abutment flank of a first latching element (5a, 5b) bears against a counterpart abutment of a tooth of the tooth arrangement (3), in which the abutment flank of a second latching element (5a, 5b) does not bear against a counterpart abutment of a tooth of the tooth arrangement (3) and in a second state the abutment flank of the second latching element (5a, 5b) bears against a counterpart abutment of a tooth of the tooth arrangement (3), in which the abutment flank of the first latching element (5a, 5b) does not bear against a counterpart abutment of a tooth of the tooth arrangement (3).

13. A dose metering device (1) according to one of the preceding claims wherein the second spacing (A2) is less than the first spacing (A1) or the first spacing (A1) is less than the second spacing (A2).

14. A dose metering device (1) according to one of the preceding claims wherein the rotation-limiting elements (4) are movable radially relative to the screwthreaded rod (2).

15. A dose metering device (1) according to one of the preceding claims wherein the teeth of the tooth arrangement (3) in a first portion along the longitudinal axis of the screwthreaded rod (2) are respectively at a constant spacing from each other in the peripheral direction of the screwthreaded rod (2) and in an adjacent second portion along the longitudinal axis of the screwthreaded rod (2) they are respectively at a further constant spacing from each other in the peripheral direction of the screwthreaded rod (2), wherein the spacing of the teeth in the first portion and the spacing of the teeth in the second portion are different.

16. A dose metering device (1) for the metered administration of an injectable product from an injection device comprising:
- a screwthreaded rod (2) with a peripherally extending guide groove (11) in the form of a screwthread and a multiplicity of engagement elements (12) which are provided in particular along a line parallel to the longitudinal axis of the screwthreaded rod (2);
- a first rotation-limiting element (4) which is connected to the injection device or a housing portion of the injection device and which in particular completely surrounds the screwthreaded rod (2), with a guide element (13) on the inside of the rotation-limiting element (4) for engagement into the guide groove (11) and a tooth arrangement (14) on the inside of the rotation-limiting element (4) for latching engagement with the engagement elements (12), wherein the teeth of the tooth arrangement (14), that are adjacent in the peripheral direction of the rotation-limiting element (4) and are arranged at the same position in the longitudinal direction of the first rotation-limiting element (4), are at a first spacing (A1) from each other in the peripheral direction; and
- a second rotation-limiting element (4) which is connected to a rotary sleeve of the injection device and which in particular completely surrounds the screwthreaded rod (2), with a guide element (13) on the inside of the rotation-limiting element (4) for engagement into the guide groove (11) and a tooth arrangement (14) on the inside of the rotation-limiting element (4) for latching engagement with the engagement elements (12), wherein the teeth of the tooth arrangement (14), that are adjacent in the peripheral direction of the rotation-limiting element (4) and are arranged at the same position in the longitudinal direction of the second rotation-limiting element (4), are at a first spacing (A1) from each other in the peripheral direction;
wherein the tooth arrangement (14) of the rotation-limiting elements (4) is of such a configuration or arrangement that in a rotary movement of the screwthreaded rod (2) relative to at least one of the rotation-limiting elements (4) by less than the first spacing (A1) at least one of the engagement elements (12) can latch in an intermediate position with the tooth arrangement (14).

17. A dose metering device (1) according to the preceding claim wherein the tooth arrangement (14) comprises at least two and in particular two, three, four, five or six partial tooth arrangements (14a, 14b) which are arranged in mutually displaced relationship in the longitudinal direction of the rotation-limiting element (4), wherein the teeth of the adjacent partial tooth arrangements (14a, 14b), that are adjacent in the longitudinal direction of the rotation-limiting element (4), are arranged in mutually displaced relationship in the peripheral direction of the rotation-limiting element (4), in particular by a spacing corresponding to half or a third or a quarter or a fifth or a sixth of the first spacing (A1).

18. A dose metering device (1) according to one of the two preceding claims wherein the tooth arrangement (14) is provided over the entire length of the rotation-limiting elements (4).

19. A dose metering device (1) according to one of the three preceding claims wherein the teeth of the tooth arrangement (14) in a first portion along the longitudinal axis of the rotation-limiting element (4) are respectively at a constant spacing from each other in the peripheral direction of the rotation-limiting element (4) and in an adjacent second portion along the longitudinal axis of the rotation-limiting element (4) they are respectively at another constant spacing from each other in the peripheral direction of the rotation-limiting element (4), wherein the spacing of the teeth in the first portion and the spacing of the teeth in the second portion are different.

20. An injection device having a dose metering device (1) according to one of the preceding claims.

## Revendications

1. Dispositif de dosage (1) pour une administration dosée d'un produit injectable à partir d'un dispositif d'injection comprenant :
- une tige filetée (2) dotée d'un filetage s'étendant dans le sens périphérique et supportant une denture (3), les dents de la denture adjacentes sur la périphérie de la tige filetée (2), le long du pas de filetage, comprenant un premier intervalle (A1) entre elles dans le sens périphérique ;
- un premier élément de limitation de rotation (4) relié au dispositif d'injection ou à une partie de boîtier du dispositif d'injection, doté d'au moins un élément d'enclenchement (5a, 5b), en particulier un, deux, trois ou quatre éléments d'enclenchement (5a, 5b) destinés à être enclenchés avec la denture (3) de la tige filetée (2) ;
- un deuxième élément de limitation de rotation (4) relié à un manchon rotatif du dispositif d'injection doté d'au moins un élément d'enclenchement (5a, 5b), en particulier deux, trois ou quatre éléments d'enclenchement (5a, 5b) destinés à être enclenchés avec la denture (3) de la tige filetée (2) ;
dans lequel
le au moins un élément d'enclenchement (5a, 5b) des éléments de limitation de rotation (4) est conçu ou disposé de façon à ce que, lors d'une rotation de la tige filetée (2) par rapport à au moins un des éléments de limitation de rotation (4), le au moins un élément d'enclenchement (5a, 5b) puisse s'enclencher de moins du premier intervalle (A1) dans une position intermédiaire.

2. Dispositif de dosage (1) selon la revendication 1, dans lequel les dents adjacentes dans le sens longitudinal de la tige filetée (2) sont disposées de façon décalée les une par rapport aux autres dans la direction périphérique, en particulier d'un intervalle correspondant à la moitié du premier intervalle (A1).

3. Dispositif de dosage (1) selon l'une des revendications précédentes, dans lequel le au moins un élément de limitation de rotation (4) comprend n éléments d'enclenchement (5a, 5b) et les dents adjacentes dans le sens longitudinal de la tige filetée (2) sont disposées de façon décalée les unes par rapport aux autres dans la direction périphérique de la n-ième partie du premier intervalle (A1).

4. Dispositif de dosage (1) selon la revendication 1, dans lequel les dents adjacentes dans le sens longitudinal de la tige filetée (2) sont disposées dans la direction périphérique à une position identique.

5. Dispositif de dosage (1) selon l'une des revendications précédentes, dans lequel au moins un des éléments de limitation de rotation (4) comprend deux éléments d'enclenchement (5a, 5b) et les pointes d'engagement (6a, 6b) des éléments d'enclenchement (5a, 5b) sont distantes les unes des autres dans la direction périphérique de la tige filetée, l'intervalle étant écarté d'un décalage prédéfini d'un multiple entier du premier intervalle (A1)

6. Dispositif de dosage (1) selon l'une des revendications précédentes, dans lequel au moins un des éléments de limitation de rotation (4) comprend n éléments d'enclenchement (5a, 5b) et les pointes d'engrènement (6a, 6b) des éléments d'enclenchement (5a, 5b) sont distantes les unes des autres dans la direction périphérique de la tige filetée, l'intervalle étant écarté d'un décalage prédéfini d'un multiple entier du premier intervalle (A1) et le décalage correspondant à la n-ième partie du premier intervalle (A1).

7. Dispositif de dosage (1) selon l'une des revendications précédentes, dans lequel le au moins un élément d'enclenchement (5a, 5b) comprend au moins deux éléments d'engrènement (7a, 7b), en particulier deux, trois, ou quatre, les éléments d'engrènement (7a, 7b) comprenant chacun un intervalle constant entre les éléments d'engrènement.

8. Dispositif de dosage (1) selon l'une des revendications précédentes, dans lequel le au moins un élément d'enclenchement (5a, 5b) comprend n éléments d'engrènement (7a, 7b), les éléments d'engrènement (7a, 7b) comprenant chacun un intervalle constant entre les éléments d'engrènement et l'intervalle entre les éléments d'engrènement correspondant à la n-ième partie du premier intervalle (A1).

9. Dispositif de dosage (1) selon l'une des revendications précédentes, dans lequel au moins deux des dents de la denture (3) adjacentes dans le sens longitudinal de la tige filetée (2) comprennent un deuxième intervalle (A2) entre elles dans le sens longitudinal.

10. Dispositif de dosage (1) selon la revendication précédente, dans lequel au moins un des éléments de limitation de rotation (4) comprend au moins deux éléments d'enclenchement (5a, 5b) et les éléments d'enclenchement (5a, 5b) sont distants dans le sens de la longueur de la tige filetée (2) du deuxième intervalle (A2).

11. Dispositif de dosage (1) selon l'une des revendications précédentes, dans lequel les éléments d'enclenchement (5a, 5b) sont formés de telle sorte qu'ils s'étendent dans le sens longitudinal de la tige filetée (2) le long d'au moins deux dents, en particulier le long de deux, trois, quatre ou n dents de la denture (3) de la tige filetée (2).

12. Dispositif de dosage (1) selon l'une des revendications précédentes, dans lequel le décalage entre les éléments d'enclenchement (5a, 5b) est conçu de telle sorte que, dans un premier état du dispositif de dosage (1), le flanc de butée d'un premier élément d'enclenchement (5a, 5b) est en contact avec une contre-butée d'une dent de la denture (3), dans lequel le flanc de butée d'un deuxième élément d'enclenchement (5a, 5b) n'est pas en contact avec une contre-butée d'une dent de la denture (3) et, dans un deuxième état, le flanc de butée du deuxième élément d'enclenchement (5a, 5b) est en contact avec la contre-butée d'une dent de la denture (3), dans lequel le flanc de butée du premier élément d'enclenchement (5a, 5b) n'est pas en contact avec une contre-butée d'une dent de la denture (3).

13. Dispositif de dosage (1) selon l'une des revendications précédentes, dans lequel le deuxième intervalle (A2) est plus petit que le premier intervalle (A1) ou le premier intervalle (A1) étant plus petit que le deuxième intervalle (A2).

14. Dispositif de dosage (1) selon l'une des revendications précédentes, dans lequel les éléments de limitation de rotation (4) sont disposés de façon mobile radialement par rapport à la tige filetée (2).

15. Dispositif de dosage (1) selon l'une des revendications précédentes, dans lequel les dents de la denture (3) comprennent un intervalle constant entre elles dans la direction périphérique de la tige filetée (2), respectivement dans une première section formée le long de l'axe longitudinal de la tige filetée (2) et comprennent un autre intervalle constant entre elles dans la direction périphérique de la tige filetée (2), respectivement dans une deuxième section adjacente formée le long de l'axe longitudinal de la tige filetée (2), l'intervalle des dents dans la première section et l'intervalle des dents dans la deuxième section étant différents.

16. Dispositif de dosage (1) pour une administration dosée d'un produit injectable à partir d'un dispositif d'injection comprenant :
- une tige filetée (2) dotée d'une rainure de guidage (11) périphérique en forme de filetage et une multitude d'éléments d'engrènement (12) formés en particulier le long d'une ligne parallèle à l'axe longitudinal de la tige filetée (2) ;
- un premier élément de limitation de rotation (4) entourant la tige filetée (2), en particulier complètement, et relié au dispositif d'injection ou à une partie de boîtier du dispositif d'injection, avec un élément de guidage (13) formé sur le côté intérieur de l'élément de limitation de rotation (4) pour un engrènement dans la rainure de guidage (11) et une denture (14) formée sur le côté intérieur de l'élément de limitation de rotation (4) pour un enclenchement avec les éléments d'engrènement (12), les dents de la denture (14) adjacentes dans la direction périphérique de l'élément de limitation de rotation (1), disposées à la même position dans le sens longitudinal du premier élément de limitation de rotation (4) comprenant un premier intervalle (A1) entre elles dans une direction périphérique;
- un deuxième élément de limitation de rotation (4) entourant la tige filetée (2), en particulier complètement, et relié à un manchon rotatif du dispositif d'injection, avec un élément de guidage (13) formé sur le côté intérieur de l'élément de limitation de rotation (4) pour un engrènement dans la rainure de guidage (11) et une denture (14) formée sur le côté intérieur de l'élément de limitation de rotation (4) pour un enclenchement avec les éléments d'engrènement (12), les dents de la denture (14) adjacentes dans la direction périphérique de l'élément de limitation de rotation (1), disposées à la même position dans le sens longitudinal du deuxième élément de limitation de rotation (4) comprenant un premier intervalle (A1) entre elles dans une direction périphérique ;
dans lequel
la denture (14) des éléments de limitation de rotation (4) est conçue ou disposée de telle sorte que, lors d'une rotation de la tige filetée (2) par rapport à au moins un des éléments de limitation de rotation (4), au moins un des éléments d'engrènement (12) puisse s'enclencher avec la denture de moins du premier intervalle (A1) dans une position intermédiaire.

17. Dispositif de dosage (1) selon la revendication précédente, dans lequel la denture (14) se compose d'au moins deux, en particulier deux, trois, quatre, cinq ou six dentures partielles (14a, 14b) disposées de façon décalée les unes par rapport aux autres dans le sens longitudinal de l'élément de limitation de rotation (4), les dents adjacentes des dentures partielles adjacentes (14a, 14b) dans le sens longitudinal de l'élément de limitation de rotation (4) étant disposées de façon décalée les unes par rapport aux autres dans la direction périphérique de l'élément de limitation de rotation (4), en particulier d'un intervalle correspondant à la moitié ou au tiers ou au quart ou au cinquième ou au sixième du premier intervalle (A1).

18. Dispositif de dosage (1) selon l'une des deux revendications précédentes, dans lequel la denture (14) est formée sur la longueur totale des éléments de limitation de rotation (14).

19. Dispositif de dosage (1) selon l'une des trois revendications précédentes, dans lequel les dents de la denture (14) comprennent chacune entre elles un intervalle constant dans la direction périphérique de l'élément de limitation de rotation (4) dans une première section formée le long de l'axe longitudinal de l'élément de limitation de rotation (4) et comprennent chacune un autre intervalle constant entre elles dans la direction périphérique de l'élément de limitation de rotation (4), dans une deuxième section adjacente formée le long de l'axe longitudinal de l'élément de limitation de rotation (4), l'intervalle des dents dans la première section et l'intervalle des dents dans la deuxième section étant différents.

20. Dispositif d'injection doté d'un dispositif de dosage (1) selon l'une des revendications précédentes.
